# EUROPEAN PATENT APPLICATION

(11) **EP 3 673 897 A1**
(43) Date of publication of application: **01.07.2020**
(21) Application number: 18849101.3
(22) Date of filing: 23.08.2018
(51) Int. Cl.: A61K 9/10, A61K 9/14, A61K 31/5415, A61K 47/10, A61K 47/28, A61K 47/32, A61K 47/36, A61K 47/38, A61K 47/40, A61K 47/42

(54) **INJECTABLE PHARMACEUTICAL COMPOSITION CONTAINING MELOXICAM, AND PREPARATION METHOD THEREFOR**

(30) Priority: 24.08.2017 CN 201710733082
(71) Applicant: Jiangsu Hengrui Medicine Co., Ltd., Lianyungang, Jiangsu 222047 (CN)
(72) Inventor: SUN, Qiong, Lianyungang Jiangsu 222047 (CN); SHI, Haixian, Lianyungang Jiangsu 222047 (CN); LIU, Kai, Lianyungang Jiangsu 222047 (CN); LIU, Ting, Lianyungang Jiangsu 222047 (CN); SHI, Congjian, Lianyungang Jiangsu 222047 (CN); CHEN, Xinxin, Lianyungang Jiangsu 222047 (CN); CHAI, Fujuan, Lianyungang Jiangsu 222047 (CN)
(74) Representative: Regimbeau
(86) International application number: PCT/CN2018/101924
(87) International publication number: WO 2019/037757

(57) **Abstract**

An injectable pharmaceutical composition containing meloxicam, and a preparation method therefor. The pharmaceutical composition comprises meloxicam nanoparticles, a surface stabilizer, and a sedimentation inhibiting agent. The pharmaceutical composition has good stability, is not easy to settle, and is applicable to industrial large-scale production.

## Description

### FIELD OF THE INVENTION

The invention relates to the field of pharmaceutical formulation, specifically to an injectable pharmaceutical composition comprising meloxicam and a method for preparing the same.

### BACKGROUND OF THE INVENTION

Meloxicam is a highly effective non-steroidal anti-inflammatory drug for treating rheumatoid arthritis, osteoarthritis and postoperative pain. Meloxicam selectively inhibits COX-2 isoenzyme, and has potent anti-inflammatory and analgesic effects and has a low gastrointestinal response. Currently, commercially available products include oral dosage forms such as meloxicam tablet and meloxicam capsule, and meloxicam injection for intramuscular injection. The disadvantage of the oral dosage form is slow absorption due to the poor wettability and low solubility of the Active Pharmaceutical Ingredient (API) (the solubility is only 0.6 µg/mL at pH 1.2 and 4.0), thus the oral dosage form cannot be used for acute rheumatism, arthritis and pain. Although intramuscular injection improves drug absorption, the absorption rate is still limited by the solubility of the API, and it can cause local tissue pain. Therefore, increasing the solubility is the key to improving the *in vivo* pharmacokinetic parameters of meloxicam.

There are many ways to improve the solubility of meloxicam, such as nanocrystals, micelles, inclusion complexes, liposomes and the like. Among them, meloxicam nanocrystals have obvious clinical advantages, they do not require carrier materials, only require a small amount of surface stabilizer, thereby avoiding toxicity problems caused by excipients (such as hemolysis, allergic reactions and the like), and they are not restricted by encapsulation rate and drug loading capacity, and can meet the need of high-dose and high-concentration formulations. Compared with conventional meloxicam formulations, the advantage of nanocrystal is also reflected in the following aspects:
1) Nanocrystals take effect rapidly, and can be used for treating acute pain. The key to the rapid effect of the drug is that the nano-sized drug particles have high solubility, fast dissolution rate and short time to peak (only tens of minutes), while the Tmax of conventional formulations can be up to 5-10 h.
2) The nanocrystal technology significantly improves the bioavailability of meloxicam, with AUC and Cmax being 1.2 times and 1.3 times that of the equivalent-dose tablets, respectively.
3) Nanocrystals expand the indication of meloxicam. Conventional dosage forms are mainly used for treating rheumatoid arthritis, painful osteoarthritis, ankylosing spondylitis and the like, while meloxicam intravenous injection can be used for treating postoperative acute pain.
4) Nanocrystals not only break through the limitation of conventional formulations that cannot be used for acute pain, but also maintain the advantage of long-term effect of conventional formulations. The average half-life of conventional formulations is 20 h. Although the half-life of the nanocrystal drug is 12 h, the efficacy can be maintained for 24 h after intravenous injection of 15 mg and 60 mg of the nanocrystal drug.
5) Compared with opioid analgesics, meloxicam intravenous injection can avoid serious adverse reactions such as respiratory depression, nausea and vomiting, excessive sedation, mental dependence and the like. Meanwhile, meloxicam, as a COX-2 selective inhibitor, has fewer adverse effects than other non-steroidal anti-inflammatory drugs.
6) Nanocrystals reduce adverse reactions such as peptic ulcer. In addition, oral formulations can also cause severe gastrointestinal responses, with an incidence of about 1.9%-7.8%. The nanocrystal drug is swallowed by macrophages after injection, and enriched in liver, spleen and lung tissues, avoiding the binding of the free drug to COX receptors on normal tissues such as the gastrointestinal tract and platelets, thereby reducing the side effects of conventional formulations.

The nanocrystal is a suspension wherein the solid particles are at the nanometer level. It is a thermodynamically unstable system that is prone to sedimentation, resulting in the injection unusable. CN101175481A discloses an injectable tacrolimus nanoparticle formulation, wherein at least one surface stabilizer is added, so that the nanoparticle size is basically maintained and the aggregation rarely occurs, when the composition is dispersed in a biologically relevant medium.

US9345665 discloses a meloxicam nanoparticle injection comprising nanoscale meloxicam particles, a surface stabilizer, and a sugar or buffer, and the like, which can reduce the sedimentation produced in the nanoparticle injection. However, there are still some insoluble particles after the injection is placed for 1 to 3 months, which will affect the stability of the injection.

Therefore, it is necessary to develop a meloxicam nanoparticle injection for clinical use with a stable quality.

### SUMMARY OF THE INVENTION

In an aspect, the present invention provides a stable meloxicam nanoparticle injection. Specifically, the present invention provides an injectable pharmaceutical composition comprising meloxicam nanoparticles and a surface stabilizer, and further comprising a sedimentation inhibitor.

Wherein, the sedimentation inhibitor can be selected from the group consisting of polyols and high-molecular polymers, such as one or more of glycerol, propylene glycol, polyethylene glycol (for example, polyethylene glycol 300, polyethylene glycol 400, polyethylene glycol 600, polyethylene glycol 4000), albumin, hydroxyethyl starch, sodium carboxymethyl cellulose and hydroxypropyl-β-cyclodextrin, preferably one or more of glycerol, polyethylene glycol and hydroxyethyl starch, and more preferably glycerol. The sedimentation inhibitor is mainly used to improve the stability of the system during storage and prevent the aggregation and precipitation of meloxicam particles.

The weight ratio of meloxicam to the sedimentation inhibitor is 1:0.1-1:100, preferably 1:0.1-1:50, more preferably 1:0.5-1:20, and most preferably 1:0.5-1:10.

In some embodiments, the surface stabilizer can be a non-ionic, anionic, cationic or zwitterionic compound or surfactant, such as one or more of polyvinylpyrrolidone, polyvinyl alcohol, hydroxypropyl methylcellulose, Tween 80, poloxamer, polyethylene glycol 15-hydroxystearate, lecithin, sodium deoxycholate, sodium cholate, sodium dodecyl sulfonate and sodium dodecyl sulfate.

Useful non-ionic surface stabilizers include, but are not limited to, hydroxypropyl methylcellulose (HPMC), polyvinylpyrrolidone, poloxamer, Tween-80, polyethylene glycol 15-hydroxystearate.

Useful anionic surface stabilizers include, but are not limited to, dioctyl sodium sulfosuccinate (DOSS), sodium dodecyl sulfonate, sodium dodecyl sulfate (SDS), docusate sodium, sodium cholate and sodium deoxycholate.

Useful cationic surface stabilizers include, but are not limited to, polymers, biopolymers, poly-N-methylpyridinium, pyridinium chloride sulfate, cationic phospholipids, chitosan, polylysine, polyvinylimidazole, polystyrene, polymethyl methacrylate trimethylammonium bromide (PMMTMABr), hexadecyltrimethylammonium bromide (HDMAB) and polyvinylpyrrolidone-2-dimethylaminoethyl methacrylate dimethyl sulfate.

Useful zwitterionic surface stabilizers include, but are not limited to, proteins, phospholipids, zwitterionic polymers and zwitterionic surfactant molecules, such as phosphatidylcholine, lecithin, gelatin and the like.

The surface stabilizer does not comprise glycerol.

Wherein, the weight ratio of meloxicam to the surface stabilizer can be 1:0.01-1:100, preferably 1:0.01-1:50, more preferably 1:0.05-1:5, and most preferably 1:0.1-1:1.

In some preferred embodiments, the surface stabilizer comprises a first surface stabilizer and a second surface stabilizer, wherein the first surface stabilizer can be a non-ionic or zwitterionic surface stabilizer, and can be selected from the group consisting of polyvinylpyrrolidone, polyvinyl alcohol, Tween 80, poloxamer, polyethylene glycol 15-hydroxystearate, lecithin and the like, and preferably polyvinylpyrrolidone, poloxamer or Tween 80; the second surface stabilizer can be an anionic surface stabilizer, and can can be selected from the group consisting of sodium deoxycholate, sodium cholate, sodium dodecyl sulfonate and sodium dodecyl sulfate, and preferably sodium deoxycholate or sodium cholate.

Wherein, the weight ratio of meloxicam to the first surface stabilizer can be 1:0.01-1:100, preferably 1:0.01-1:50, more preferably 1:0.05-1:5, and most preferably 1:0.1-1:1.

Wherein, the weight ratio of meloxicam to the second surface stabilizer can be 1:0.01-1:100, preferably 1:0.01-1:50, more preferably 1:0.01-1:5, and most preferably 1:0.01-1:1.

In some preferred embodiments, the preferred combination comprises a first surface stabilizer that is polyvinylpyrrolidone, a second surface stabilizer that is sodium deoxycholate, and a sedimentation inhibitor that is one or more selected from the group consisting of glycerol, polyethylene glycol and hydroxyethyl starch, and preferably glycerol.

The average particle size of the meloxicam nanoparticles of the present invention is less than 2000 nm, for example less than 1500 nm, preferably less than 1000 nm, more preferably less than 500 nm, and most preferably less than 200 nm.

The injectable pharmaceutical composition of the present invention can also comprise a liquid medium selected from the group consisting of water, saline solution, vegetable oil (such as safflower seed oil) and organic solvent (such as ethanol, *t*-butanol, hexane and ethylene glycol) and the like, and preferably water.

In the pharmaceutical composition, meloxicam is present in an amount of 10-100 mg/mL, preferably 10-50 mg/mL, more preferably 15-35 mg/mL, and most preferably 25 mg/mL.

In the pharmaceutical composition, the amount of the sedimentation inhibitor is 0.1-100 mg/mL, preferably 0.1-50 mg/mL, and more preferably 1-20 mg/mL.

In the pharmaceutical composition, the amount of the first surface stabilizer is 0.1-100 mg/mL, preferably 1-50 mg/mL, and more preferably 1-20 mg/mL; and the amount of the second surface stabilizer is 0.1-100 mg/mL, preferably 1-50 mg/mL, and more preferably 1-10 mg/mL.

In another aspect, the present invention also provides an injectable pharmaceutical composition comprising: (1) meloxicam nanoparticles, (2) polyvinylpyrrolidone, (3) sodium deoxycholate, (4) a sedimentation inhibitor and (5) water,
wherein, the sedimentation inhibitor is one or more selected from the group consisting of glycerol, polyethylene glycol and hydroxyethyl starch, and preferably glycerol; the average particle size of meloxicam nanoparticles is less than 500 nm, and preferably less than 200 nm; the weight ratio of meloxicam to the sedimentation inhibitor is 1:0.5-1:20, and preferably 1:0.5-1:10; the weight ratio of meloxicam to polyvinylpyrrolidone is 1:0.05-1:5, and preferably 1:0.1-1:1; and the weight ratio of meloxicam to sodium deoxycholate is 1:0.05-1:5, and preferably 1:0.1-1:1.

In another aspect, the present invention also provides a method for preparing an injectable meloxicam pharmaceutical composition, comprising steps of:
1) mixing a surface stabilizer, meloxicam and an optional sedimentation inhibitor;
2) grinding the above mixed system to prepare a dispersion; and optionally
3) mixing a sedimentation inhibitor with the above dispersion.

The grinding apparatus suitable for the present invention includes a disperse mill (such as a ball mill, attrition mill and vibration mill) and medium mill (such as a sand mill and bead mill). These disperse mills are well known in the art.

Although the addition of a surface stabilizer to the meloxicam nanoparticle injection composition can improve the dispersibility of meloxicam nanoparticles during the preparation process, the nanoparticle formulation is a suspension liquid that is a thermodynamically unstable system, aggregation and sedimentation will occur during long-term storage due to the Ostwald ripening phenomenon, which hinders the clinical application of the nanoparticle formulation. In the present invention, the addition of a sedimentation inhibitor such as glycerol to the composition can increase the density or viscosity of the solution, inhibit the sedimentation of meloxicam particles, thereby improving the stability of meloxicam nanoparticles during long-term storage and facilitating its clinical application.

In the specification and claims of the present application, unless otherwise indicated, the scientific and technical terms used herein have the meanings generally understood by a person skilled in the art. However, in order to understand the present invention better, definitions and explanations of some related terms are provided below.

The expression in the present invention, for example "average particle size less than 2000 nm", means that the average particle size value of at least 50% by weight of the active material particles is less than about 2000 nm.

The average particle size of the particles of the present invention can be measured by conventional particle size measurement techniques well known to the person skilled in the art. Such techniques include, for example, sedimentation field flow fractionation, photon correlation spectroscopy, light scattering and the like.

The term "weight-to-volume ratio" in the present invention refers to the weight (g) of the ingredient per 100 mL of the liquid system, i.e. g/100mL.

The term "D10" in the present invention refers to the corresponding particle size when the cumulative particle size distribution percentage of a sample reaches 10%. The term "D50" refers to the corresponding particle size when the cumulative particle size distribution percentage of a sample reaches 50%. The term "D90" refers to the corresponding particle size when the cumulative particle size distribution percentage of a sample reaches 90%.

"Optional" or "optionally" means that the event or circumstance described subsequently can, but need not, occur, and such a description includes the situation in which the event or circumstance does or does not occur. For example, "optionally comprise (comprising) a sedimentation inhibitor" means that a sedimentation inhibitor can be, but need not be, present, and such a description includes the situation wherein the sedimentation inhibitor is present and the situation wherein the sedimentation inhibitor is not present.

### DETAILED DESCRIPTION OF THE INVENTION

The present invention is further described in detail by the following examples and experimental examples. These examples and experimental examples are for illustrative purposes only, and are not intended to limit the scope of the present invention.

In the following examples, the insoluble particles in the nanoparticle injection were determined with reference to the microscopic method under the general rule of United States Pharmacopeia (USP) <788>. Each test sample container contained no more than 3,000 particles with a particle size of 10 µm or more, and no more than 300 particles with a particle size of 25 µm or more.

The impurity amount of the nanoparticle injection was determined by HPLC. Detection condition: ODS-2 column (5 µm, 4.6 × 150 mm), mobile phase: methanol/water, detection wavelengths: 260 nm and 350 nm.

### Example 1

Polyvinylpyrrolidone was used as the first surface stabilizer, sodium deoxycholate was used as the second surface stabilizer, and glycerol was used as the sedimentation inhibitor to prepare the nanoparticle injection. The specific prescription ingredients and their dosages are as follows:

**Table 1 Prescription ingredients and dosages**

| Name | Dosage (weight-to-volume ratio) | Prescription ingredients of each injection | Prescription amount |
|---|---|---|---|
| Meloxicam | 2.5% | 30 mg | 150 g |
| PVP-K17 | 0.5% | 6 mg | 30 g |
| Sodium deoxycholate | 0.25% | 3 mg | 15 g |
| Glycerol | 2.5% | 30 mg | 150 g |
| Water | qs | qs | qs |

Preparation method:
1) a prescription amount of PVP-K17 and sodium deoxycholate were dissolved in 50% of the total weight of water;
2) the above system was added with the API meloxicam, and mixed well;
3) the above mixed system was added to the cavity of a mill filled with grinding beads, and grinded for 6 hours; and
4) the above grinded solution was added with the sedimentation inhibitor, and set to the target weight.

The indicators such as particle size, pH, osmotic pressure, insoluble particles and related substances of the above nanoparticle injection were determined. The test results are as follows:

**Table 2 Results of particle size, pH, osmotic pressure and insoluble particles**

| Osmotic pressure | Time | pH | Average particle size (nm) | PdI | Insoluble particles (number) | |
|---|---|---|---|---|---|---|
| | | | | | ≥10 µm | ≥25 µm |
| 302 mOsm/kg | Initial point | 7.02 | 86.80 | 0.264 | 17 | 10 |
| | 40°C 10d | 6.84 | 93.10 | 0.244 | 16 | 9 |

**Table 3 Results of related substances**

| Time | Maximum single impurity | Total impurities |
|---|---|---|
| Initial point | 0.132% | 0.324% |
| 40d_ 10D | 0.164% | 0.367% |

The results showed that the injection prepared with 2.5% glycerol as the sedimentation inhibitor had a qualified osmotic pressure, and there were no significant changes in pH, particle size, insoluble particles and related substances after the injection was placed under an accelerated condition of 40°C for a period of time, indicating that the sample had a good stability.

### Example 2

PVP-K17 was used as the first surface stabilizer, sodium cholate was used as the second surface stabilizer, and glycerol was used as the sedimentation inhibitor to prepare the nanoparticle injection. The specific prescription ingredients and their dosages are as follows:

**Table 4 Prescription ingredients and dosages**

| Name | Dosage (weight-to-volume ratio) | Prescription ingredients of each injection | Prescription amount |
|---|---|---|---|
| Meloxicam | 2.5% | 30 mg | 150 g |
| PVP-K17 | 0.5% | 6 mg | 30 g |
| Sodium deoxycholate | 0.25% | 3 mg | 15 g |
| Glycerol | 5% | 60 mg | 300 g |
| Water | qs | qs | qs |

Preparation method:
1) a prescription amount of PVP-K17 and sodium cholate were dissolved in 50% of the total weight of water;
2) the above system was added with the API meloxicam, and mixed well;
3) the above mixed system was added to the cavity of a mill filled with grinding beads, and grinded for 8 hours; and
4) the above grinded solution was added with the sedimentation inhibitor, and set to the target weight.

The indicators such as particle size, pH, osmotic pressure, insoluble particles and related substances of the above nanoparticle injection were determined. The test results are as follows:

**Table 5 Results of particle size, pH, osmotic pressure and insoluble particles**

| Osmotic pressure | Time | pH | Average particle size (nm) | PdI | Insoluble particles (number) | |
|---|---|---|---|---|---|---|
| | | | | | ≥10 µm | ≥25 µm |
| 621 mOsm/kg | Initial point | 7.01 | 86.03 | 0.256 | 14 | 7 |
| | 40°C 10 days | 6.79 | 92.10 | 0.243 | 17 | 11 |

**Table 6 Results of related substances**

| Time | Maximum single impurity | Total impurities |
|---|---|---|
| Initial point | 0.138% | 0.343% |
| 40°C 10 days | 0.152% | 0.328% |

The results showed that the injection prepared with 5% glycerol as the sedimentation inhibitor had a qualified osmotic pressure, and there were no significant changes in pH, particle size, insoluble particles and related substances after the injection was placed under an accelerated condition of 40°C for a period of time, indicating that the sample had a good stability.

### Example 3

The ability of different sedimentation inhibitors to inhibit the sedimentation of nanoparticle composition was determined by observing the appearance. The tested nanoparticle compositions comprised 2.5% of meloxicam, 0.5% of PVP-K17 and 0.25% of sodium deoxycholate by weight-to-volume ratio, as well as different types and dosages of sedimentation inhibitors (see Table 7). The nanoparticle injection was prepared by the same preparation method as in Example 1. The test results are shown in Table 7 below.

**Table 7 Inhibition of sedimentation by different sedimentation inhibitors**

| Type and dosage of sedimentation inhibitor | Appearance under different placement conditions | | |
|---|---|---|---|
| | 40°C 1M | 25°C 1M | 2-8°C 1M |
| 5% of mannitol | Small amount of precipitation | Small amount of precipitation | Good |
| 10% of mannitol | Small amount of precipitation | Small amount of precipitation | Good |
| 5% of sucrose | Good | Small amount of precipitation | Good |
| 10% of sucrose | Small amount of precipitation | Small amount of precipitation | Good |
| 30% of sucrose | Precipitation | Precipitation | Good |
| 10% of dextran 40 | Precipitation | Precipitation | Good |
| 10% of glycerol | Good | Good | Good |
| 20% of glycerol | Good | Good | Good |

The results showed that temperature had a certain affect on the stability of the product. The higher the temperature, the more unstable the system. In addition, glycerol had a better inhibition effect on precipitation than other sedimentation inhibitors.

### Example 4

The effect of different types of sedimentation inhibitors was determined by detecting insoluble particles in the samples. The tested nanoparticle compositions comprised 2.5% of meloxicam, 0.5% of PVP-K17 and 0.25% of sodium deoxycholate by weight-to-volume ratio, as well as different types and dosages of sedimentation inhibitors (see Table 8). The test results of insoluble particles after the samples were placed at 40°C for 15d and 1M are shown in Table 8 below.

**Table 8 Effect of different sedimentation inhibitors on insoluble particles of the product**

| Type and dosage of sedimentation inhibitor | 40°C 15d insoluble particles (number) | | 40°C 1M insoluble particles (number) | |
|---|---|---|---|---|
| | ≥10 µm | ≥25 µm | ≥10 µm | ≥25 µm |
| 10% of sucrose | 121 | 18 | 197 | 30 |
| 30% of sucrose | 110 | 14 | Obvious sedimentation | Obvious sedimentation |
| 10% of dextran 40 | 123 | 48 | 97 (yellow solid on the filter membrane) | 10 (yellow solid on the filter membrane) |
| 5% of hydroxy ethyl starch | 59 | 16 | 57 | 20 |
| 10% of glycerol | 75 | 7 | 70 | 7 |
| 20% of glycerol | 64 | 4 | 53 | 13 |
| 100 mM disodium hydrogen phosphate | 107 | 25 | Obvious sedimentation | Obvious sedimentation |
| 10% of sucrose +100 mM disodium hydrogen phosphate | 171 | 28 | Obvious sedimentation | Obvious sedimentation |

The results showed that when sucrose, dextran 40 or phosphate buffer was used as the sedimentation inhibitor, the product showed an increase in insoluble particles and sedimentation after placed under an accelerated condition for 1M. When glycerol was used as the sedimentation inhibitor, the number of insoluble particles was small, and the nanoparticle system had a good stability.

### Example 5

The effect of different sedimentation inhibitors on the stability of the products was determined. The tested nanoparticle compositions comprised 2.5% of meloxicam, 0.5% of PVP-K17 and 0.25% of sodium deoxycholate by weight-to-volume ratio, as well as different types and dosages of sedimentation inhibitors (see Table 9). The test results of pH, particle size and insoluble particles after the samples were placed at 40°C or 60°C for 10d are shown in Table 9 below. The appearance results after the samples were placed at room temperature (25°C) for 1M are shown in Table 10.

**Table 9 Results of pH, particle size and insoluble particles**

| Sedimentation inhibitor | Time | pH | Average particle size | PdI | Insoluble particles (number) | |
|---|---|---|---|---|---|---|
| | | | | | ≥10 µm | ≥25 µm |
| 5% of glycerol | Initial point | 7.01 (23.4°C) | 86.03 nm | 0.256 | 14 | 7 |
| | 40°C 10d | 6.79 (26.2°C) | 92.10 nm | 0.243 | 17 | 11 |
| | 60°C 10d | 6.85 (25.0°C) | 98.56 nm | 0.235 | 28 | 16 |
| 2.5% of glycerol | Initial point | 7.02 (23.5°C) | 86.80 nm | 0.264 | 17 | 10 |
| | 40°C 10d | 6.84 (26.4°C) | 93.10 nm | 0.244 | 16 | 9 |
| | 60°C 10d | 6.86 (25.7°C) | 99.98 nm | 0.232 | 13 | 8 |
| 2.5% of mannitol | Initial point | 7.03 (24.0°C) | 86.44 nm | 0.267 | 11 | 6 |
| | 40°C 10d | 6.93 (26.6°C) | 93.58 nm | 0.252 | 17 | 10 |
| | 60°C 10d | 6.88 (26.1°C) | 98.81 nm | 0.239 | 21 | 13 |

**Table 10 Appearance of the samples comprising different sedimentation inhibitors after placed at room temperature for 1M**

| Sedimentation inhibitor | Time | Appearance |
|---|---|---|
| 5% of glycerol | Room temperature, 1M | Light yellow emulsion, no sedimentation and good appearance |
| 2.5% of glycerol | Room temperature, 1M | Light yellow emulsion, no sedimentation and good appearance |
| 2.5% of mannitol | Room temperature, 1M | Light yellow emulsion with sedimentation and yellow flaky solid at the bottom |

The results showed that 1) when 5% of glycerol, 2.5% of glycerol or 2.5% of mannitol was used as the sedimentation inhibitor, there was little change in pH after the samples were placed at 40°C or 60°C for 10d; 2) for the sample comprising 5% of glycerol, 2.5% of glycerol or 2.5% of mannitol, there was little change in insoluble particles after the samples were placed at 40°C for 10d; for the sample comprising 5% of glycerol or 2.5% of mannitol, there was a slightly increase in insoluble particles after the samples were placed at 60°C for 10d, but there was no significant change for the sample comprising 2.5% of glycerol; and 3) after the samples were placed at room temperature for 1M, there were flaky crystals at the bottom of the sample with 2.5% of mannitol as the sedimentation inhibitor, but the appearance of the sample with glycerol as the sedimentation inhibitor was good. The product with glycerol as the sedimentation inhibitor has a better stability than the product with 2.5% of mannitol as the sedimentation inhibitor.

### Example 6

The nanoparticle injection obtained in Example 1 was placed respectively at (25°C±2°C, RH60±5%) and (2-8°C) for 6 months to determine its stability. The results are shown in Tables 11 and 12.

**Tables 11 Results of the accelerated test**

| | Time (month) | | |
|---|---|---|---|
| | 0 | 3 | 6 |
| Impurity amount (%) | 0.21 | 0.26 | 0.28 |
| pH | 7.0 | 7.1 | 7.0 |
| Particle size | 100.4 | 110.7 | 112.6 |
| Amount (%) | 102.9 | 103.7 | 104.5 |

**Tables 12 Results of the long-term test**

| Tested factor | Time (month) | | |
|---|---|---|---|
| | 0 | 3 | 6 |
| Impurity amount (%) | 0.21 | 0.22 | 0.24 |
| pH | 7.0 | 7.1 | 7.0 |
| Particle size | 100.4 | 134.0 | 103.5 |
| Amount (%) | 102.9 | 103.5 | 104.1 |

The results showed that there was no significant change in the property, pH, particle size and impurity amount of the sample after long-term placement under each condition, and the stability of the sample was good.

## Claims

1. An injectable pharmaceutical composition, comprising meloxicam nanoparticles and a surface stabilizer, **characterized in that** the pharmaceutical composition further comprises a sedimentation inhibitor, wherein the sedimentation inhibitor is one or more selected from the group consisting of glycerol, propylene glycol, polyethylene glycol, albumin, hydroxyethyl starch, sodium carboxymethyl cellulose and hydroxypropyl-β-cyclodextrin, preferably one or more of glycerol, polyethylene glycol and hydroxyethyl starch, and more preferably glycerol.

2. The pharmaceutical composition according to claim 1, **characterized in that** the weight ratio of meloxicam to the sedimentation inhibitor is 1:0.1-1:100, preferably 1:0.1-1:50, more preferably 1:0.5-1:20, and most preferably 1:0.5-1:10.

3. The pharmaceutical composition according to claim 1, **characterized in that** the surface stabilizer is one or more selected from the group consisting of polyvinylpyrrolidone, polyvinyl alcohol, hydroxypropyl methylcellulose, Tween 80, poloxamer, polyethylene glycol 15-hydroxystearate, lecithin, sodium deoxycholate, sodium cholate, sodium dodecyl sulfonate and sodium dodecyl sulfate.

4. The pharmaceutical composition according to claim 1, **characterized in that** the weight ratio of meloxicam to the surface stabilizer can be 1:0.01-1:100, preferably 1:0.01-1:50, more preferably 1:0.05-1:5, and most preferably 1:0.1-1:1.

5. The pharmaceutical composition according to claim 1, **characterized in that** the surface stabilizer does not comprise glycerol.

6. The pharmaceutical composition according to claim 1, **characterized in that** the surface stabilizer comprises a first surface stabilizer and a second surface stabilizer, wherein the first surface stabilizer is selected from the group consisting of non-ionic surface stabilizers and zwitterionic surface stabilizers, preferably polyvinylpyrrolidone, polyvinyl alcohol, hydroxypropyl methylcellulose, Tween 80, poloxamer, polyethylene glycol 15-hydroxystearate or lecithin, and more preferably polyvinylpyrrolidone, poloxamer or Tween 80; the second surface stabilizer is an anionic surface stabilizer, preferably sodium deoxycholate, sodium cholate, sodium dodecyl sulfonate or sodium dodecyl sulfate, and more preferably sodium deoxycholate or sodium cholate.

7. The pharmaceutical composition according to claim 6, **characterized in that** the weight ratio of meloxicam to the first surface stabilizer is 1:0.01-1:100, preferably 1:0.01-1:50, more preferably 1:0.05-1:5, and most preferably 1:0.1-1:1; and the weight ratio of meloxicam to the second surface stabilizer is 1:0.01-1:100, preferably 1:0.01-1:50, more preferably 1:0.01-1:5, and most preferably 1:0.01-1:1.

8. The pharmaceutical composition according to claim 1, **characterized in that** the average particle size of the meloxicam nanoparticles is less than 2000 nm, preferably less than 1000 nm, more preferably less than 500 nm, and most preferably less than 200 nm.

9. The pharmaceutical composition according to claim 1, **characterized in that** the pharmaceutical composition further comprises a liquid medium selected from the group consisting of water, saline solution, safflower seed oil, ethanol, *t*-butanol, hexane and ethylene glycol, and preferably water.

10. The pharmaceutical composition according to claim 9, **characterized in that** meloxicam is present in an amount of 10-100 mg/mL, preferably 10-50 mg/mL, more preferably 15-35 mg/mL, and most preferably 25 mg/mL, based on the weight-to-volume ratio of the active ingredient to the pharmaceutical composition.

11. An injectable pharmaceutical composition, comprising: (1) meloxicam nanoparticles, (2) polyvinylpyrrolidone, (3) sodium deoxycholate, (4) a sedimentation inhibitor and (5) water,
wherein, the sedimentation inhibitor is one or more selected from the group consisting of glycerol, polyethylene glycol and hydroxyethyl starch, and preferably glycerol; the average particle size of the meloxicam nanoparticles is less than 500 nm, and preferably less than 200 nm.

12. The pharmaceutical composition according to claim 11, **characterized in that** the weight ratio of meloxicam to the sedimentation inhibitor is 1:0.5-1:20, and preferably 1:0.5-1:10; the weight ratio of meloxicam to polyvinylpyrrolidone is 1:0.05-1:5, and preferably 1:0.1-1:1; and the weight ratio of meloxicam to sodium deoxycholate is 1:0.05-1:5, and preferably 1:0.1-1:1.

13. A method for preparing the injectable pharmaceutical composition according to claims 1 to 12, comprising steps of:
1) mixing a surface stabilizer, meloxicam and an optional sedimentation inhibitor;
2) grinding the above mixed system to prepare a dispersion; and optionally
3) mixing a sedimentation inhibitor with the above dispersion.
